# EUROPEAN PATENT APPLICATION

(11) **EP 0 890 368 A1**
(43) Date of publication of application: **13.01.1999**
(21) Application number: 98202240.2
(22) Date of filing: 02.07.1998
(51) Int. Cl.: A61M 1/16

(54) **Blood treatment apparatus**

(30) Priority: 09.07.1997 IT MI971632
(71) Applicant: SIS-TER S.p.A., 26020 Palazzo Pignano (Cremona) (IT)
(72) Inventor: Porro, Giampiero, 22100 Como (IT); Erba, Giuseppe, 20052 Monza (Milano) (IT)
(74) Representative: Dragotti, Gianfranco

(57) **Abstract**

An integrated apparatus for the treatment of blood adapted for dialysis applications and based on the use of a membrane exchange device (30) is described.

The exchange device is in particular formed by an outer jacket (40), which is an integral and permanent part of the apparatus and is made from a biocompatible material; one or a plurality of cavities, in which respective filter cartridges (20) can be inserted and then removed for the treatment of a patient, are provided in the jacket (40).

This apparatus makes it possible to re-use the filter cartridges (20) for the same patient, after the jacket (40) has been disinfected, with substantial advantages from both the economic and medical points of view.

In a preferred embodiment, these cartridges are obtained from dialysis filters conventionally available in commerce with their outer cylindrical casing removed.

## Description

The present invention relates to an apparatus for the treatment of blood using a membrane exchange device.

In the following description reference is made, unless otherwise specified, to filters for extracorporeal haemodialysis in order to indicate membrane exchange devices, although it will be appreciated that this is no more than a non-limiting example, since these devices are also used in other current dialysis techniques such as haemodiafiltration, ultrafiltration, plasma exchange, haemoperfusion, haemoconcentration and in the heat and gas exchange typical of applications in the cardio-pulmonary sector.

All haemodialysis filters are at present designed to be used in haemodialysis apparatus in combination with a set of tubular members and various accessories commonly known in overall terms as a dialysis line and these components (filter and dialysis line) are usually provided in separate sterile packages so that different types of filter most suitable for the specific treatment required by the patient can be used; they are also designed for single use and are also commonly called disposable.

A conventional dialysis filter is essentially formed by a bundle of hollow fibres, within which the blood to be purified flows during the dialysis treatment; this bundle has at both of its ends respective caps provided with connectors for connection to the haematic circuit and is also provided with appropriate seals of the O-ring type, which may or may not be integral with the caps, to allow a hydraulic seal between the latter and the end heads of the fibre bundle.

The fibre bundle is enclosed in a cylindrical casing of plastic material, provided with two connectors for connection to the dialysis solution circuit, the fibre bundle being secured in a leak-tight manner to this casing by means of a resin which is usually polyurethane-based.

In practice, the apparatus for dialysis treatment that are currently in use substantially comprise:
(1) a tubular segment (arterial line) which connects the dialysis filter to an artery or to the arterio-venous fistula of the patient, a pump, generally of peristaltic type, to take the blood from the patient and supply it in a pressurised manner to the filter via one of its end caps;
(2) the dialysis filter;
(3) a tubular segment (venous line) for the output of the purified blood from the filter, so that it can be returned to a vein of the patient or to the arterio-venous fistula, this line being connected to the other end cap of the filter and incorporating, among other things, the venous chamber (which is adapted to retain any air bubbles or clotting);
(4) a line for supplying dialysis solution to one of the two connectors provided on the outer casing of the filter, this supply taking place under the action of a pump provided in this line;
(5) a discharge line for the dialysis solution.

It has been observed in recent years that the dialysis solution can be readily contaminated by bacteria and bacterial fragments, for instance as a result of faulty hygiene in plant for treating the water needed to prepare these dialysis fluids or in centralised distribution plant: these bacteria may also be found in concentrated dialysis solutions and lastly may be concealed in the hydraulic circuit of the apparatus. The species most frequently found in dialysis fluid are the *Pseudomonas* and since these bacteria are relatively large and therefore retained by the membrane of the dialysis filter, they are not potentially harmful to the patient but if the bacteria grow and move, they release fragments, such as endotoxins in the case of gram-negative bacteria, from their cell membrane.

These endotoxins are lipopolysaccharide complexes formed by three main components, the smallest of which has a molecular weight in the range 650-5000 and cannot therefore be retained by simple filtration by the capillary membranes of dialysis filters.

If these endotoxin fragments pass into the blood, they can cause acute febrile reactions and can in the long-term play a part in causing chronic complications.

For this reason, it has become current practice to use capillary membrane filters which are known as expressly designed safety filters, in which the dialysis solution, before reaching the actual dialysis assembly, is subject to a treatment which combines the filtration mechanism with an adsorption mechanism in order to provide a microbiologically pure dialysis fluid as output.

This filter (an example of which is the filter produced and marketed under the name DIASAFE by FRESENIUS MEDICAL CARE) is periodically subject to disinfectant treatments using chemical agents and/or the action of heat.

The cleaning treatment for this filter is usually concomitant with the treatment to clean and disinfect the dialysis apparatus (hydraulic circuit for the preparation of the dialysis bath).

It will be appreciated that this practice, which should as a rule be applied after each dialysis treatment, affects the productivity of a dialysis ward as the dialysis apparatus cannot be used during the disinfection cycle.

Only two dialysis sessions are considered to be possible with two staff shifts of 6.5 hours; reducing the time needed for disinfection would make it possible to carry out three dialysis sessions.

The reduction, where possible, of health costs is also a trend common to all countries, including those that are highly industrialised; the re-use of dialysis filters has, for this reason, already been attempted and is common practice in some countries, although this technique has given rise to considerable debate not only as regards problems connected with safety but also with many other aspects.

As regards aspects of biocompatibility, the performance of a filter that has already been used is better than that of a new filter; during the first treatment, i.e. using a new filter, the surfaces of the fibres of the filter in contact with the blood tend to be covered by a thin protein layer, called a "biofilm", as a result of which the activation of the "complement system" and therefore the reactions of the patient's immune system tend to be substantially reduced during dialysis treatments subsequent to the first (i.e. when the filter is being re-used).

On the other hand, the possible reduction of the efficiency or "clearance" capacity (the term normally used to designate operating efficiency) of a filter that has already been used, caused by the interposition of a further resistance to the dialysis exchange, tends to be of little significance given the high performance of the fibres used for the manufacture of those filters currently available in commerce.

It is evident from the brief notes given above that it would be desirable to have an integrated blood treatment system capable of:
- making use of the proven positive aspects of the re-use technique in relation to the tubular fibres of the dialysis filter;
- integrating the safety filtration assembly contained in the dialysis solution circuit;
- integrating specialised filtration assemblys for specific dialysis techniques in a single device;
- simplifying the connections of the dialysis fluid circuit and the blood line in the case of non-standard dialysis techniques that therefore require several filtration assemblys in series with one another;
- enabling more efficient and more rapid washing and disinfectant treatment of the dialysis apparatus, with particular reference to heat sterilisation;
- reducing, in overall terms, the costs of dialysis treatment, even when dialysis filters are not re-used, by reducing production costs.

These objects are achieved by the integrated blood treatment apparatus according to the present invention, of the type comprising a membrane exchange device in which a flow path for the blood and a flow path for an exchange fluid are defined, a line for the connection of this device with an artery of the patient, this line being provided with a pump adapted to take the blood from the patient and supply it in a pressurised manner to the relative path of the membrane exchange device, an output line for the blood purified by the above device so that it can be returned to a vein of the patient, a line for supplying the exchange fluid to the relative path of the device, in which a pump is provided to carry out this supply operation, and a line for the discharge of the exchange fluid, characterised in that the membrane exchange device comprises a substantially hollow jacket which is an integral and permanent component of the apparatus and is internally accessible for the introduction and removal of a filter cartridge, this hollow jacket being made from a biocompatible material and shaped so that it has at least one inner cavity which, in addition to forming a housing for the cartridge, defines on opposite sides thereof at least one chamber for the distribution and/or collection of the exchange fluid.

In accordance with a preferred embodiment of the present invention, the hollow jacket is formed by two semi-cylindrical valves that can be moved between a closed position in which they form said at least one inner cavity and an open or parted position in which the filter cartridge, which essentially comprises a filter of conventional type with no outer casing, can be inserted into or removed from the above-mentioned cavity.

In this preferred embodiment of the invention, each valve of the hollow jacket comprises two semi-cylindrical, adjacent portions of parallel axis, and at least one of the dialysis fluid distribution and/or collection chambers is formed in the connection zone between these two adjacent portions, as a result of which the reciprocal closure of the two valves concomitantly forms either said two cylindrical cavities and said at least one chamber for the distribution and/or collection of the dialysis fluid.

As discussed above, the configuration of the two valves is such that they fit the cylindrical shapes of the filter cartridges, without their relative casings, thereby obtaining with the two end caps the required seal and the same performance of a conventional filter cartridge.

In this manner, part of the membrane exchange device becomes an integral part of the dialysis apparatus whose non-removable members, represented by the valves, may be made from plastics materials (preferably a technopolymer, such as polysulphone) resistant to prolonged use and possibly to the heat disinfection temperature, or from a metal such as stainless steel. It will be appreciated that, in contrast to current practice, these non-removable members are made from more valuable and therefore more costly materials but only not this does not increase the cost of the dialysis apparatus and treatments, but also enables substantial savings to be made.

As mentioned above, the membrane exchange device of the apparatus of the present invention uses a modified filter cartridge, in the sense that use is made of the same cartridge known up to now, without its outer cylindrical casing.

This means, in the first place, that at the end of a dialysis treatment it is possible to remove the filter cartridges (i.e., in the simplest configuration, the safety filter and the dialysis filter) and to disinfect the dialysis apparatus in a rapid and highly efficient manner so that a subsequent dialysis treatment for another patient can be commenced very rapidly after new sterile cartridges have been installed.

The washing and disinfecting operations for the filter cartridges that have just been used can be carried out off line on that part of the cartridge, i.e. the bundle of tubular fibres, that is not only the most costly member but is also the member that gives rise to the formation of the "biofilm" mentioned above.

These operations can be carried out with procedures and apparatus of the type commonly used in those countries in which the re-use technique is most widespread; the cartridges may then be labelled and stored so that they can be re-used for a dialysis treatment of the same patient, with whom a certain number of cartridges can therefore be associated, entailing obvious advantages not only from the economic point of view but also from the point of view of preventing the transmission of any pathogenic agents from one patient to another. In other words, this eliminates the main obstacle to the technique of re-use.

Secondly, the technology used to produce the filter cartridge does not meet substantial and major modifications, especially as regards the fitting together of the bundle of tubular fibres with the end caps and with the O-ring seals and "potting" with resin of medical quality which is therefore biocompatible and much more costly than the polyurethane resins commonly used for industrial applications.

Specialised filter cartridges for various dialysis and other applications can be achieved using bundles of capillary fibres obtained from appropriate materials and with appropriate dimensional characteristics as is commonly already the case; the dialysis filter market can, for instance, be divided into three segments based on price, performance and different fibres:
- filters with (low-flux) cellulose-based fibres;
- filters with high-flux cellulose-based and low-flux synthetic-based fibres;
- filters with high-flux synthetic-based fibres.

The filter cartridge without its casing is preferably produced with a covering on the exterior of the tubular bundle that is adapted to protect the sterility and mechanical integrity of the fibres and is made of an appropriate removable or, for instance, hydrosoluble material.

This protective covering can then be used each time that the used filter cartridge is cleaned and disinfected for re-use.

In addition to these advantages, the present invention also makes it possible to provide the hollow jacket with a structure such that a fluid-dynamic path for the exchange fluid can be provided in order to optimise mass transport, minimising so-called "shunt" phenomena and enabling the dialysis fluid to flow from one filter cartridge to another to carry out a different function.

The invention also makes it possible to achieve, in addition to the counter-current fluid-dynamic scheme typically used for dialysis, the cross-flow scheme characterising the overwhelming majority of applications in the cardio-pulmonary sector in which, within the filtration assemblys, the blood to be treated flows outside the bundle of fibres, while the interior of the fibres is traversed by exchange fluids such as oxygen and water.

In the cardio-pulmonary sector, filters exclusively of a disposable type are produced using technologies absolutely identical to those used for dialysis filters: they can therefore be designed and produced in the same way as the dialysis cartridges discussed above.

In this case the membrane exchange device comprising the two valves forms an integral part of the "heart-lung" apparatus and, in the same way as in the dialysis application, these valves form a hollow jacket which can in turn be accessed for the insertion and removal of the cartridges with the functions of "oxygenator", "heat exchanger" and "haemoconcentrator".

The following detailed description of the overall invention, setting out its characteristic features and the advantages deriving therefrom, is given purely by way of non-limiting example, with reference to the accompanying drawings, in which:
Fig. 1 is an axonometric view of a filter cartridge of conventional type normally used in dialysis;
Fig. 2 is a similar view of the previous cartridge without its outer casing;
Fig. 3 is an axonometric schematic view, with some parts removed, of a membrane exchange device which is part of the apparatus of this invention, shown in an operating condition during which dialysis is not being carried out;
Figs. 4 and 5 show respective axonometric and front views of the device of the preceding Figure in an operating condition ready for connection with the remainder of the apparatus of the invention;
Fig. 6 shows the device of Figs. 3-5, with a part removed in order provide a view of its interior;
Figs. 7 to 9 are views corresponding to those of Figs. 3 to 5, relating to a second embodiment of the membrane exchange device of this invention;
Fig. 10 is a cross-section along the line X-X of Fig. 8;
Figs. 11, 12 and 13 are respective diagrams showing the operation of corresponding embodiments of apparatus of the invention to which the exchange device of Figs. 3 to 5 is applied.

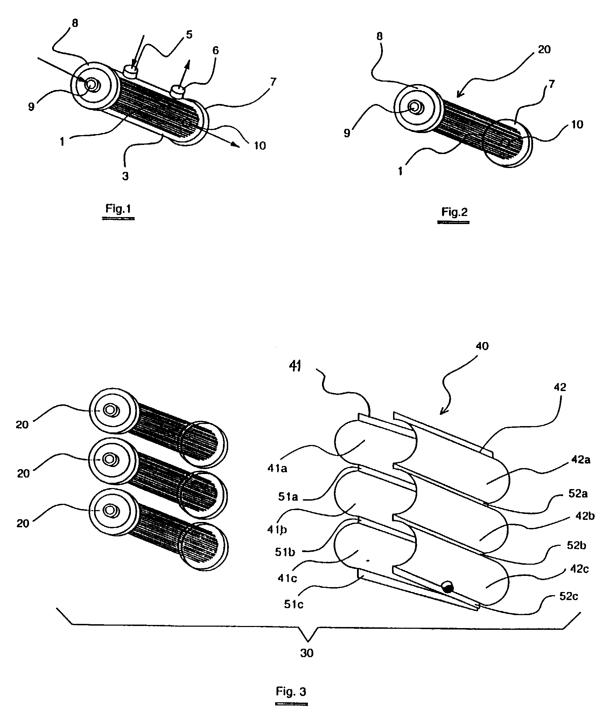

As mentioned above, Fig. 1 shows a conventional filter of the type already mentioned above and used in dialysis apparatus; it comprises a bundle 1 of hollow fibres disposed inside a cylindrical housing 3 of plastic material comprising two connectors 5 and 6 for the input and output connection of the filter with the hydraulic circuit of the dialysis fluid.

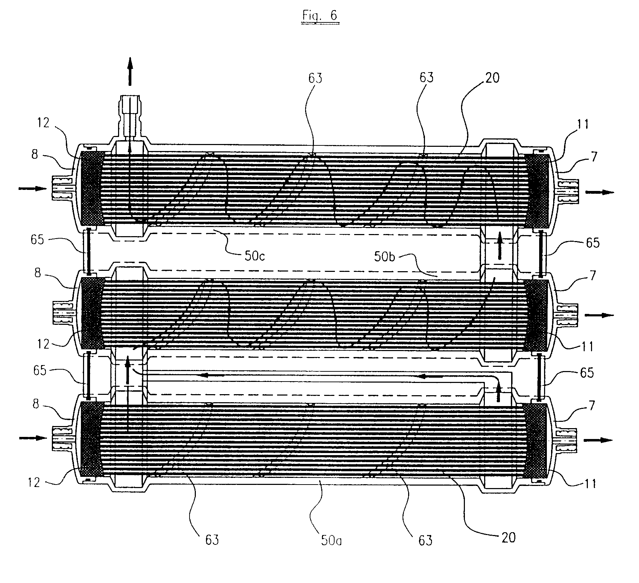

The cylindrical casing 3 is closed at its ends by two caps 7 and 8, each of which is provided with a connector, 9 and 10 respectively, for the input and output connection of the filter with the haematic circuit; for this purpose, the filters are generally provided with gaskets (not shown in the Figures and generally formed by O-rings) providing a seal between the caps and the planes of the two end heads 11 and 12 (shown in Fig. 6) of the fibre bundle.

Fig. 2 shows a filter cartridge 20 of the present invention; in this Figure, components structurally or functionally equivalent to those already shown with respect to the conventional filter bear the same reference numerals.

A synoptic comparison of this and the preceding Figure shows that the filter cartridge 20 is in practice equivalent to a conventional filter without its outer casing; operating methods are also similar, as will be explained below: it should be noted, however, that as there is no outer casing, the caps 7 and 8 are connected solely to the heads 11 and 12 of the fibre bundle.

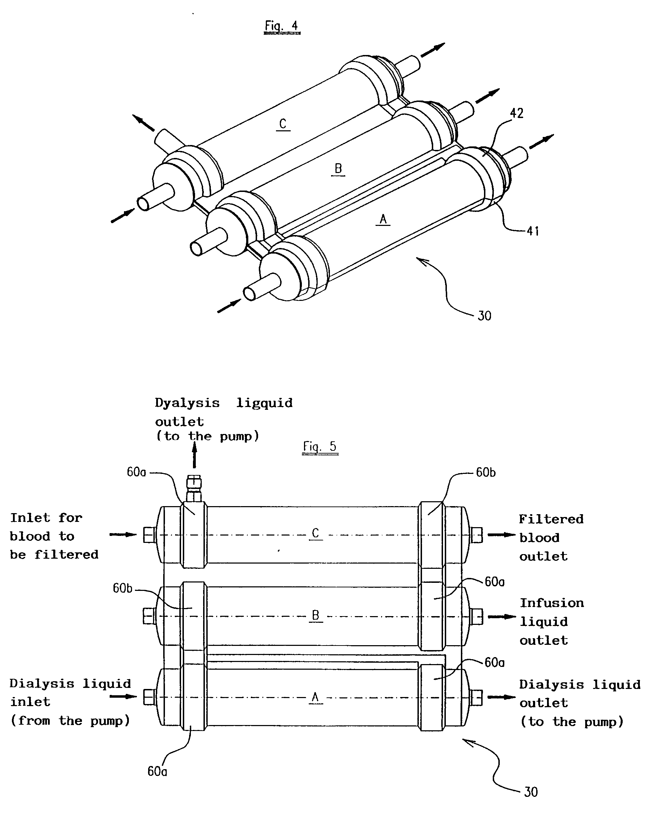

The apparatus of the invention further comprises a membrane exchange device which is formed in this case by a filtration assembly shown overall by 30 and 130 in the drawings; respective embodiments of this assembly are shown in Figures 4-6 and Figs. 7-10 and are described in detail below.

The first of these assemblies comprises a jacket 40 formed by two valves 41 and 42 which are mutually hinged or otherwise connected so as to allow one or a plurality of filter cartridges 20 to be inserted in the jacket, as shown in Fig. 3: for this purpose, each valve 41 and 42 has semi-cylindrical portions, 41a, 41b, 41c; 42a, 42b, 42c respectively, which, when the valves are juxtaposed in the jacket's closed position, define corresponding cavities 50a, 50b, 50c, for housing the filter cartridges 20.

In accordance with a preferred embodiment of the invention, respective connection zones 51a, 51b, 51c; 52a, 52b, 52c, which define corresponding chambers for the distribution 60b and collection 60a of the dialysis fluid, are then provided between one semi-cylindrical portion and an adjacent portion of the relative valve 41 and 42.

The embodiment of the filtration assembly shown in Figs. 4-6 is of the type wherein the blood flows in a counter-current relationship with the flow of dialysis fluid; in order, however, to promote the exchange between the blood and the dialysis fluid, ribs that divert the flow of the blood such that it moves, within each cavity 50a-50c, along a helical path, are disposed within the semi-cylindrical portions 41a-41c and 42a-42c.

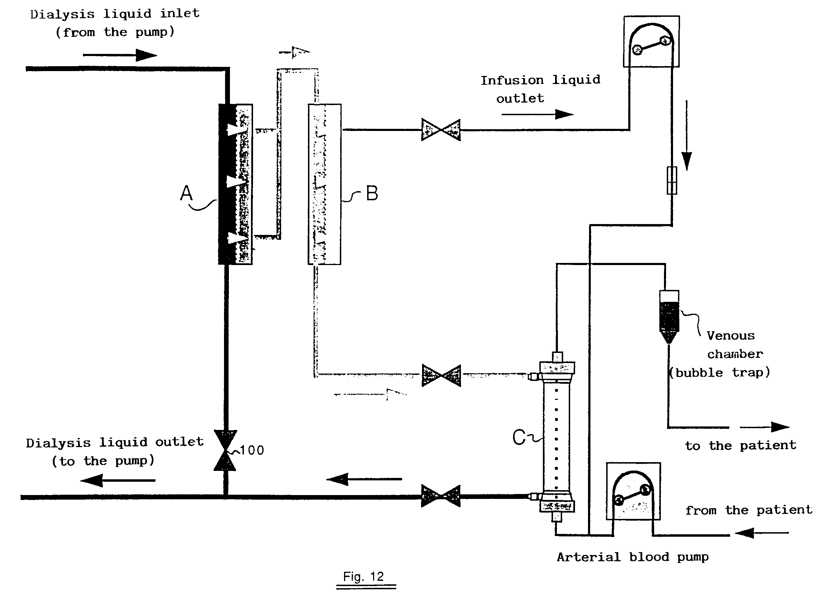

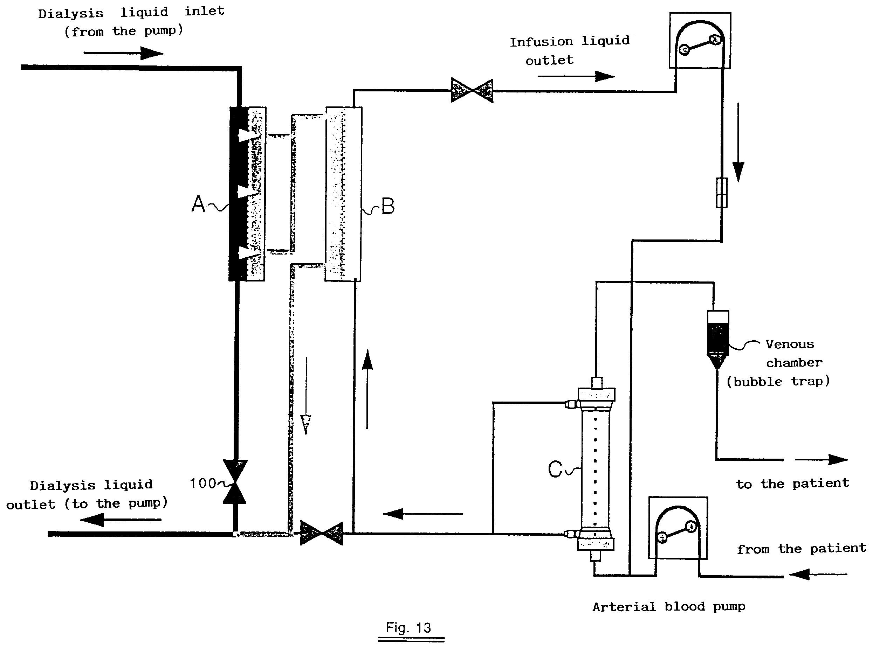

The circulation of the various fluids passing through the filtration assembly 30 is shown by arrows and some captions in Figs. 5 and 6, and is also shown diagrammatically, in relation to the overall apparatus, in Figs. 11-13 which show the relative circuits with some of their components; in these Figures, the individual filtration assemblies forming part of the assembly 30 are shown by the letters A, B and C.

The first of these assemblies is connected as input to a dialysis fluid circulation pump (not shown); within this assembly, the flow of fluid from the pump may be split depending on whether a valve 100 downstream of the assembly A is open or closed.

If this valve is open, part of the dialysis fluid therefore returns to the pump while the other part goes on to a successive filtration circuit.

In any case, the dialysis fluid that is filtered by the assembly A flows into the collection chambers 60a and reaches the adjacent distribution chamber 60b of the second filtration assembly B.

Here the flow of dialysis fluid circulating in the filter assembly 30 is further divided into a portion that is not filtered and another portion that, once filtered, is adapted to provide the infusion fluid or infusion; in this case, however, in contrast to the assembly A, it is the portion of filtered fluid that is directed out of the assembly 30, while the residual portion passes into the collection chamber 60a and reaches the adjacent distribution chamber 60b of the third filter assembly C.

The infusion fluid may then, depending on the case, be combined with the filtered blood output from the assembly C and then supplied to the patient, and/or may be added to the blood that is supplied to this filter assembly by the arterial blood pump:
this will depend on the configuration of the dialysis apparatus and the treatment to be administered to the patient; these possible alternatives are shown in the diagrams of Figs. 11-13.

The filter assembly 30 lastly comprises gaskets 65 providing a seal between the two valves 41 and 42; the material from which these valves are made must, as mentioned above, be strong, able to withstand heat disinfection temperatures and biocompatible:
a plastic material (the polysulphone mentioned above) or a stainless steel is therefore to be preferred. Lastly, the apparatus shown in the diagrams of Figs. 11-13 is supplemented by certain known components, including means for dispersing any air bubbles in the filtered blood to be returned to the patient (the "venous chamber") and an arterial blood circulation pump.

The results that can be achieved with the apparatus of the invention are readily evident from the above description of its more structural and functional aspects, and have been discussed before: reference should therefore be made in this respect to the arguments set out above.

It should be stressed here that the teaching deriving from the invention should not be considered to be limited solely to dialysis apparatus but also extends, as mentioned at the beginning of this description, to all apparatus for the treatment of blood comprising a membrane exchange device.

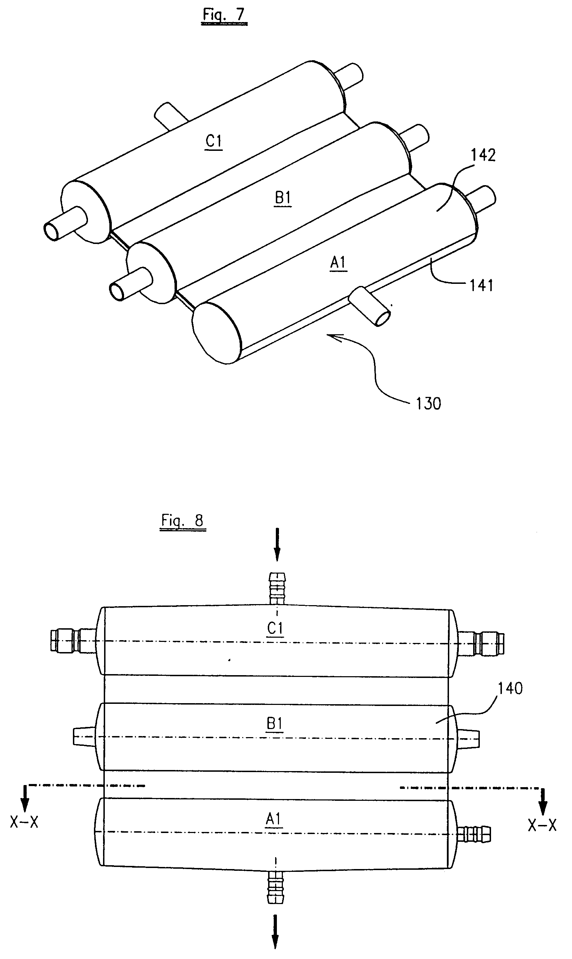

Reference may be made in this respect to the variant of this device, with respect to the filter assembly 30, shown in Figs. 7 to 10, where structural components corresponding to those already discussed in the previous embodiment bear the same reference numerals prefixed by 100; in view of the analogies between the two cases, the following description of this embodiment will be shorter and intended essentially to show the most important differences with respect to what has already been explained.

This variant relates to a filter assembly 130 for cardio-pulmonary applications, in which the blood passing through it flows transverse to the flow of fluids, whether fluid or gaseous, with which it interacts by means of the filter cartridges 120 and circulates outside the bundle of capillary fibres in a different manner with respect to dialysis filters: i.e. using the cross-flow scheme commonly used for these applications.

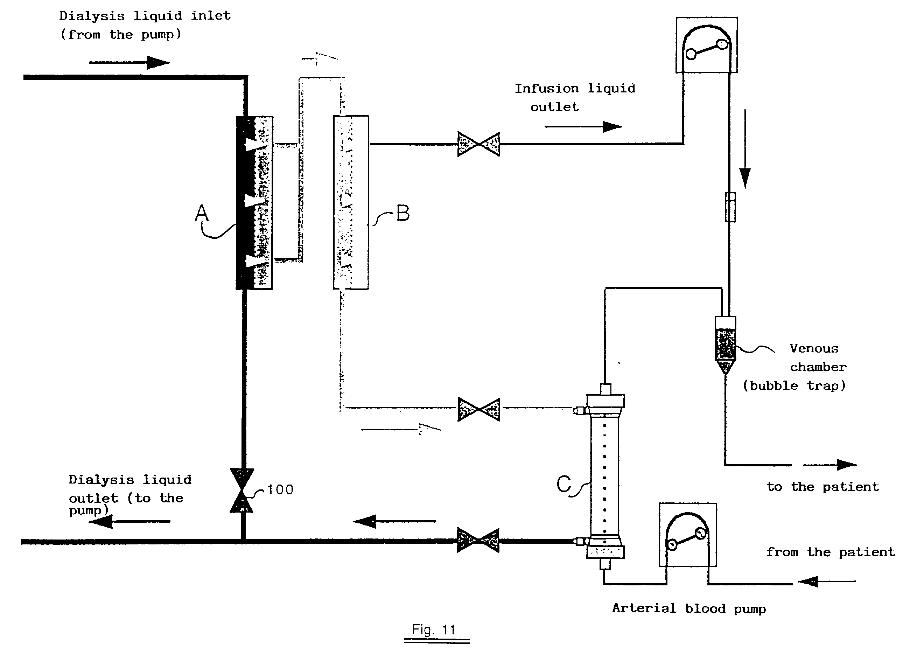

The assembly 130 is formed by three filtration assemblys A1, B1, C1, each of which comprises a filter cartridge 120 provided with an axial inner channel 121 through which water, gas, or ultrafiltrate fluid flows, as shown by the captions of Fig. 10 and in accordance with the procedures for this type of treatment.

In this case as well, the filter assembly 130 is provided with an outer jacket 140 formed by two valves 141 and 142 hinged or connected together in another equivalent way, so that they can be opened or closed to enable the insertion or removal of the cartridge 120; each valve then comprises the semi-cylindrical portions (not numbered in Figs. 7-10; for details of these, reference can, however, be made to the description of Fig. 3) which, when the valves 141 and 142 are in the position closing the jacket, define corresponding cavities adapted to house the filter cartridges 120.

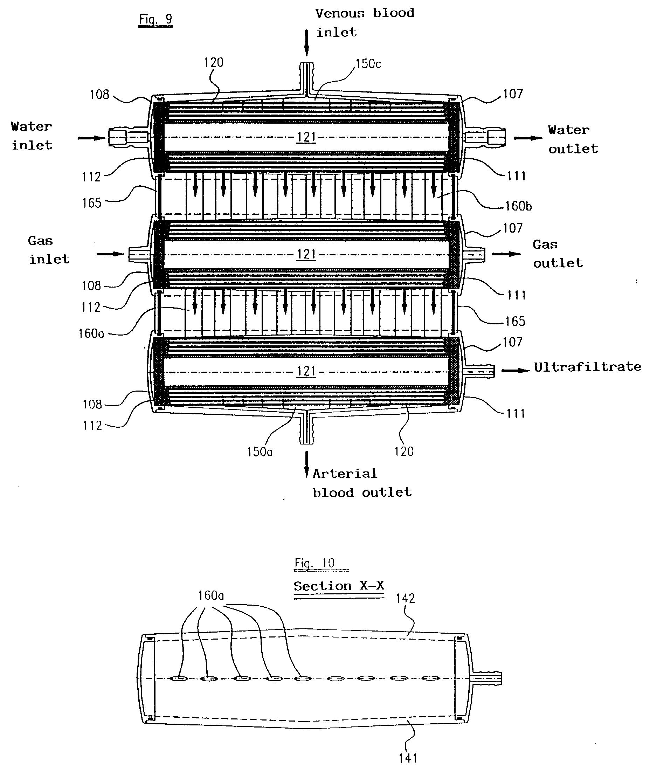

Differently from the dialysis filtration assembly 30, in this variant each of these cavities communicates with the adjacent cavity or cavities by means of a series of passages 160a and 160b, of substantially elliptical cross-section, formed by the juxtaposition of corresponding recesses provided (see Fig. 10) in each valve 141 and 142 when they are closed; the blood flows through these passages as shown by the arrows of Fig. 9.

Although explanatory diagrams of the remainder of the apparatus in which the filtration assembly 130 is inserted have not been provided as in the preceding case, reference may nevertheless be made to the normal components of conventional cardio-pulmonary apparatus.

As will be appreciated from the brief description given here, this alternative embodiment of the invention formed by an apparatus for cardio-pulmonary applications provided with a membrane exchange device with a cross-flow operating scheme, also achieves the objects of the invention.

It will also be readily appreciated that the technique of re-using filter cartridges 120 as already explained in relation to dialysis, can again be used for this apparatus and, by the same token, that it is also possible to increase the yield of the apparatus by reducing the times needed to disinfect and wash it.

It should be noted in the case of cardiac surgery applications that re-use is not the appropriate term as these operations are events that generally take place once in a patient's life, but the practice is to retain blood treatment devices for short periods and in the case of serious pathologies as well as should the same patient require further surgery.

These results are due to the possibility offered by this particular membrane exchange device which has a fixed portion, i.e. the jacket formed by the two valves integrated into the apparatus itself, and a moving portion, i.e. the membrane produced in the form of filter cartridges, which may be removed and subsequently re-used.

Other variants of the invention with respect to the above description obviously cannot be excluded.

By way of example, the shape of the filter membranes, formed in the preceding embodiments by the cartridges 20 and 120, although to be preferred since these cartridges can then be obtained in practice from commercially available cartridges with all the deriving advantages (for instance the fact that existing production plant and processes can be used), is not the only shape and it will therefore be readily appreciated that variations of it may involve substantial changes to the configuration of the relative filtration assembly with the two valves forming the above-mentioned jacket.

These and any other modifications are covered by the scope of the invention as set out in this description and the accompanying claims.

## Claims

1. An integrated blood treatment apparatus comprising
- a membrane (1) exchange device (30, 130) in which a flow path for the blood and a flow path for an exchange fluid are defined,
- a line for the connection of this device (30, 130) with an artery of the patient, this line being provided with a pump adapted to take the blood from the patient and supply it in a pressurised manner to the relative path of the membrane (1) exchange device (30, 130),
- an output line for the blood purified by the above device so that it can be returned to a vein of the patient,
- a line for supplying the exchange fluid to the relative path of the exchange device (30, 130), in which a pump is provided to carry out this supply operation,
- a line for the discharge of the exchange fluid,
characterised in that the membrane (1) exchange device (30, 130) comprises an outer jacket (40, 140) which is an integral and permanent component of the apparatus and is internally accessible for the introduction and removal of a filter cartridge (20, 120), this jacket being made from a biocompatible material and shaped so that it has at least one inner cavity (50a, 50b, 50c; 150a, 150b, 150c) which, in addition to forming a housing for the filter cartridge (20, 120), defines on opposite sides thereof chambers for the distribution and/or collection (60a, 60b; 160a, 160b) of the exchange fluid.

2. An apparatus according to claim 1, wherein said at least one cavity (50a-50c; 150a-150c) is substantially cylindrical and the related filter cartridge (20, 120) essentially comprises a filter of conventional type without its outer cylindrical casing (3).

3. An apparatus according to claim 2, wherein the exchange fluid distribution and/or collection chambers (60b, 60a; 160a, 160b) are disposed on substantially opposite generatrices of the cavity (50a-50c; 150a-150c) communicating with them.

4. An apparatus according to claims 1, 2 or 3, which is adapted for dialysis applications and wherein the exchange fluid is a fluid dialysis solution.

5. An apparatus according to any of the preceding claims, wherein the outer jacket (40, 140) of the membrane (1) exchange device (30, 130) comprises two valves (41, 42; 141, 142), each of which is provided with at least one semi-cylindrical portion (41a, 41b, 41c; 42a, 42b, 42c) that can be moved between a first position in which they are juxtaposed to form said at least one cavity (50a-50c; 150a-150c) and a second position in which they are spaced so that a filter cartridge (20, 120) can be inserted in or removed from this cavity.

6. An apparatus according to claim 5, wherein each valve (41, 42; 141, 142) of the outer jacket (40, 140) comprises two adjacent semi-cylindrical portions (41a-41c; 42a-42c) with parallel axes between which there is provided a connection zone (51a, 51b, 51c; 52a, 52b, 52c), as a result of which the reciprocal juxtaposition of the two valves causes the concomitant formation of the relative cylindrical cavities (50a-50c; 150a-150c) and the exchange fluid distribution and/or collection chamber (60b, 60a; 160a, 160b) interposed therebetween.

7. An apparatus according to claims 5 and 6, wherein the valves (41, 42; 141, 142) of the outer jacket (40, 140) are hinged together.

8. An apparatus according to any of the preceding claims, wherein the biocompatible material from which the outer jacket (40, 140) is made is a metal material.

9. An apparatus according to any of claims 1 to 7, wherein the biocompatible material from which the outer jacket (40, 140) is made is a polymer material.

10. An apparatus according to claim 9, wherein the polymer material is a technopolymer such as polysulphone.

11. An apparatus according to any of the preceding claims, wherein the blood and exchange fluid flow in counter-current relationship within the relative cavity or cavities (50a-50c; 150a-150c) of the outer jacket (30, 130).

12. An apparatus according to any of claims 1 to 11, wherein the blood and exchange fluid flow as cross-flows in the relative cavity or cavities (50a-50c; 150a-150c) of the outer jacket (30, 130).

13. A filter cartridge for an apparatus according to any of the preceding claims, comprising a fibre bundle (1) provided at its opposite ends with means (7, 8, 9, 10) for its hydraulic connection to the apparatus, characterised in that it further comprises an outer removable protective covering wherein the bundle (1) and the connection means (7-10) are contained, which, once this covering has been removed, are externally exposed so that they can be introduced into the relative cavity (50a-50c; 150a-150c) of the exchange device (30, 130).
